# EUROPÄISCHE PATENTSCHRIFT

(11) **EP 3 498 206 B1**
(45) Veröffentlichungstag und Bekanntmachung des Hinweises auf die Patenterteilung: **17.01.2024**
(21) Anmeldenummer: 18191929.1
(22) Anmeldetag: 31.08.2018
(51) Int. Cl.: A61B 17/70, A61B 17/80

(54) **OKZIPITALPLATTE**
OCCIPITAL PLATE
PLAQUE OCCIPITALE

(30) Priorität: 14.12.2017 DE 102017129989
(43) Veröffentlichungstag der Anmeldung: 19.06.2019
(73) Patentinhaber: Böhm, Heinrich, Dr., 99425 Weimar (DE)
(72) Erfinder: Böhm, Heinrich, 99425 Weimar (DE); Burger, Andreas, 78532 Tuttlingen (DE); Wenzler, Klaus, 78665 Frittlingen (DE); Widmaier, Gerd, 78532 Tuttlingen (DE)
(74) Vertreter: Mammel und Maser Patentanwälte PartG mbB

(56) Entgegenhaltungen:
- EP-A1- 0 744 923
- DE-T2- 69 605 589
- US-A1- 2007 016 189
- US-A1- 2008 086 124
- US-A1- 2013 238 033
- US-A1- 2015 196 328
- US-A1- 2015 327 901

## Beschreibung

Die vorliegende Erfindung betrifft eine Okzipitalplatte und ein Set mit einer Okzipitalplatte.

Okzipitalplatten werden am menschlichen Schädel befestigt und dienen zusammen mit einem an den Halswirbeln zu befestigenden Fixationssystem zur Stabilisierung des Kopf-Halswirbelsäulenübergangs bei angeborenen und erworbenen Instabilitäten bei Verletzungen, Tumoren oder entzündlichen oder degenerativen Erkrankungen.

Da die anatomisch stabilsten Ankerpunkte für Fixationsschrauben in der Nähe der Hinterhauptsschuppe liegen, müssen die herkömmlichen Okzipitalplatten relativ weit kopfwärts platziert werden. Diese Positionierung bringt den Nachteil mit sich, dass der operative Zugang ebenfalls relativ weit nach kopfwärts geführt werden muss, wo die Bedingungen für Wundheilung und Implantatdeckung deutlich schlechter sind als 1-2 cm fußwärts. Dies bedingt, dass die Längsträger für die Ankoppelung an Ankerpunkte der Halswirbelsäule bei allen bisherigen Okzipitalplatten entweder stark gebogen werden müssen oder abwinkelbar sein müssen. Dies bedeutet neben der Verlängerung des Hebelarmes entweder eine mechanische Schwächung der Längsträger oder ein voluminöses Implantat, welches durch seinen Platzbedarf die Fixierungsmöglichkeiten am 1. und 2. Halswirbel einschränkt.

Häufig werden die Okzipitalplatten in kranial-kaudaler Orientierung am Schädel befestigt. Hierzu weisen die Okzipitalplatten einen Mittelabschnitt, der sich in kranial-kaudale Richtung erstreckt mit Durchführungen für Befestigungsmittel zum Befestigen am Schädel, auf.

Um die gewünschte Stabilisierung zu erreichen, ist eine starre Verbindung zwischen der Okzipitalplatte und der Stützeinrichtung erforderlich.

Die Stützeinrichtungen umfassen Stäbe, die mit ihrem distalen Ende an der Wirbelsäule befestigt werden.

Solche Okzipitalplatten sind beispielsweise aus der DE 10 2011 051 975 A1 bekannt, wobei die dortige Okzipitalplatte fünf Durchführungen zur Befestigung von Schrauben im Schädel aufweist. Die Platte weist einen Mittelabschnitt mit den Durchführungen und zwei Plattenflügel auf, die sich im kaudalen Endbereich des Mittelabschnitts seitlich erstrecken. Zudem sind in den beiden seitlichen Plattenflügeln und in kranialer Richtung Biegezonen vorgesehen, um die Okzipitalplatte optimal am Schädelknochen anzulegen. Auch aus der Ebene herausgebogene Plattenflügel weisen die erforderliche Stabilität bzw. Starrheit auf. An den beiden seitlichen Plattenflügeln kann jeweils ein Stab befestigt werden, dessen distales Ende an der Wirbelsäule befestigt wird. Da große Kräfte auf die Okzipitalplatte und somit auf die Befestigungsmittel wirken, ist ein Federring vorgesehen, der ein ungewolltes selbstständiges Herauslösen der Schraube aus dem Schädelknochen verhindert.

Die EP 2 370 010 B1 lehrt eine Okzipitalplatte, die eine Vielzahl an Bohrungen für Befestigungsmittel aufweist, um die Platte fest am Knochen zu befestigen. Die Befestigung solch einer Platte ist zwar stabil, allerdings muss für jede der vielen Schrauben ein Loch in den Schädel des Patienten gebohrt werden, was das Komplikationsrisiko für den Patienten und den Zeit- und Kostenaufwand steigen lässt. Die Stäbe werden mit der Okzipitalplatte über ein in einem Längsschlitz in der Okzipitalplatte vorgesehenes, verschiebbares Montageelement verschraubt.

Aus der US 2007/016189 A1 ist eine gattungsgemäße Okzipitalplatte bekannt, bei der die wenigsten eine Aufnahme eine Durchgangsbohrung zur Verbindung mit der Stützeinrichtung aufweist und wobei die Längsachse der Durchgangsbohrung im Wesentlichen parallel zu der Längsachse der Durchführung verläuft.

Aus der DE 696 05 589 T2 ist eine Okzipitalplatte mit einer Aufnahme mit einer Durchgangsbohrung für eine Stützeinrichtung bekannt.

Die EP 0 744 923 lehrt eine Okzipitalplatte, die kontinuierlich mit der benachbarten Sektion des Stabes durch ein Verbindungsteil verbunden ist. Das Verbindungsteil ist für eine Stützeinrichtung, den Stab, vorgesehen und mit dem Mittelabschnitt der Okzipitalplatte starr verbunden.

Nachteilig bei den aus dem Stand der Technik bekannten Okzipitalplatten ist, dass eine Vielzahl von Bohrungen in den Schädel erforderlich ist und dass die gesamte Last auf die Befestigungsmittel im Schädelknochen wirkt. Ein weiterer Nachteil ist die mangelnde Winkelstabilität der Verbindung zwischen Okzipitalplatte und Stabsystem.

Es ist daher die Aufgabe der vorliegenden Erfindung, eine Okzipitalplatte anzugeben, die schonender und einfacher am Schädel zu befestigen ist und sich durch eine besonders hohe Stabilität auszeichnet.

Diese Aufgabe wird durch die Merkmale des Anspruchs 1 gelöst.

Erfindungsgemäß weist die Okzipitalplatte an ihrem in kaudale Richtung weisenden Ende ein Hakenelement auf, das dazu dient, in der Hinterhauptsöffnung eingehakt zu werden. Das Hakenelement dient zur zusätzlichen Befestigung der Okzipitalplatte im Hinterhauptsloch (Foramen magnum). Die auf die Okzipitalplatte wirkende Last wird somit nicht nur über Schrauben im Schädelknochen, sondern auch über das hakenförmige Halteelement auf den Schädelknochen übertragen.

Dadurch, dass das Hakenelement in das Hinterhauptsloch eingreift und das Hinterhauptbein von unten unter- bzw. umgreift, wird eine zusätzliche Lastaufnahme bereitgestellt, ohne dass weitere Bohrungen in den Schädel erforderlich sind.

Vorzugsweise ist das Hakenelement in kranialer Richtung des Mittelabschnitts korrespondierend zu dem unteren Bereich des oberhalb des Hinterhauptslochs liegenden Hinterhauptbeins konvex, d.h. mit seiner Oberseite nach oben gekrümmt, um für den Schädelknochen eine breite Auflagefläche bereitzustellen.

Der Mittelabschnitt kann unterschiedlich lang ausgebildet sein. Je nach anatomischer Gegebenheit kann der Mittelabschnitt auch interoperativ aus kranialer Richtung gekürzt werden, so dass weniger Durchführungen für Befestigungsmittel verfügbar sind.

Die Durchführungen können alternativ auch als Gleitloch oder Langloch ausgebildet sein. Auch sind bei mehreren Durchführungen beliebige Kombinationen an unterschiedlich ausgebildeten Durchführungen möglich.

Die Okzipitalplatte weist wenigstens eine Aufnahme mit einer Durchgangsbohrung auf, mittels der die Okzipitalplatte mit der Stützeinrichtung verbindbar ist.

Erfindungsgemäß verläuft die Längsachse der Durchgangsbohrung in der Aufnahme in einer zu der Vorderseite des Mittelabschnitts parallel liegenden Ebene und rechtwinklig zur Längsachse des Mittelabschnitts.

Erfindungsgemäß sind der Mittelabschnitt, die Aufnahme und das Hakenelement einstückig ausgebildet.

Vorzugsweise wird die Okzipitalplatte mit der Stützeinrichtung als Set angeboten.

Im Rahmen der vorliegenden Erfindung umfasst die Stützeinrichtung Stabelemente, die mit ihren distalen Enden, den freien Enden, an den Wirbeln befestigt werden sowie Verbindungselemente und/oder Befestigungsmittel.

Somit kann die Okzipitalplatte mit dem erfindungsgemäßen Hakenelement entweder direkt mittels Befestigungsmitteln wie z.B. Schrauben mit den Stabelementen verbunden werden. Solche Befestigungsmittel sind aus dem Stand der Technik bekannt.

Zudem kann eine Verbindung zwischen Okzipitalplatte und Stabelement über ein Verbindungselement und Befestigungsmittel wie Schrauben erfolgen, wobei in dieser Variante das Verbindungselement vorzugsweise stabförmig ist.

Vorzugsweise weist die Innenfläche der Durchgangsbohrung in der Aufnahme der Okzipitalplatte für die Verbindung mit dem stabförmigen Verbindungselement Führungselemente auf, die komplementär zu den Konturen auf der Mantelfläche des Verbindungselements sind, so dass das Verbindungselement in die Durchgangsbohrung in der Aufnahme hineingesteckt werden kann und eine Rotationsbewegung zwischen Verbindungselement und Okzipitalplatte verhindert wird. Somit wird eine winkelstabile und formschlüssige Verbindung erhalten. Insbesondere ist das stabförmige Verbindungselement eine Welle.

In einer bevorzugten Variante weisen auch die Stabelemente an ihrem proximalen Ende eine Durchgangsbohrung auf oder sind mit einem Kopplungsmittel mit einer solchen Durchgangsbohrung fest verbunden, wobei die Innenseiten der Durchgangsbohrungen ebenfalls Führungselemente aufweisen, die zu den Konturen auf der Mantelfläche des Verbindungselements komplementär sind. Hierdurch wird eine winkelstabile und formschlüssige Verbindung zwischen Verbindungselement und den Stabelementen erzielt.

Vorzugsweise wird das Verbindungselement in die Aufnahme der Okzipitalplatte und in die Bohrung im oder am proximalen Ende des Stabelements hineingesteckt und ist somit gegen eine Rotationsbewegung gesichert und gegen eine seitliche Verschiebebewegung arretierbar, vorzugsweise durch Schrauben und eine Bohrung, die sich rechtwinklig zu der jeweiligen Durchgangsbohrung durch die Aufnahme oder am proximalen Ende des Stabelements erstreckt.

Die Verbindungselemente und Stabelemente (und ggfs. Kopplungselemente für die Stabelemente) sind in unterschiedlichen Größen und Durchmessern an der Okzipitalplatte adaptierbar.

Die Okzipitalplatte kann aus Kunststoff oder Stahl, vorzugsweise aus Titan gefertigt sein.

Die Okzipitalplatte kann mit einem oder mehreren Befestigungsmitteln am Schädel befestigt, insbesondere verschraubt werden. Über das Verbindungselement, das formschlüssig in die Aufnahme eingreift, wenn das Verbindungselement in die Aufnahme geschoben ist, wird eine winkelstabile Kopplung mit der Stützeinrichtung geschaffen.

Die spezielle Verbindung zwischen der Okzipitalplatte und den Stabelementen über das stabförmige Verbindungselement, die eine Einstellung des Winkels zwischen Okzipitalplatte und Stabelement und Winkelstabilität ermöglicht, kann im Übrigen auch ohne das Hakenelement an der Okzipitalplatte realisiert werden, auch wenn selbstverständlich die Variante mit dem Hakenelement an der Okzipitalplatte besonders bevorzugt ist.

Die Erfindung sowie weitere vorteilhafte Ausführungsformen und Weiterbildungen derselben werden im Folgenden anhand der in den Zeichnungen dargestellten Beispiele näher beschrieben und erläutert.

Es zeigen:
Figur 1 die Draufsicht auf die Sichtseite der Okzipitalplatte (von dem Schädel abgewandte Seite),
Figur 2 die Seitenansicht auf die rechte Seite der Okzipitalplatte aus Figur 1,
Figur 3 die Draufsicht auf die Rückseite (dem Schädel zugewandte Seite) der Okzipitalplatte,
Figur 4 eine perspektivische Ansicht eines stabförmigen Verbindungselements, auf das die Okzipitalplatte und das proximale Ende des Stabelements aufgeschoben sind,
Figur 5 die Seitenansicht des Verbindungselements,
Figur 6 eine Seitenansicht auf das Stabelement
   a) mit Draufsicht auf die Durchtrittsöffnung im proximalen Ende
   b) Ansicht entsprechend Pfeil A in Figur 6a,
Figur 7 einen Schnitt durch ein Stabelement, das an seinem proximalen Ende ein Kopplungselement mit einer Durchgangsbohrung aufweist,
Figur 8 eine Seitenansicht des Hinterhauptbeins, an dem die Okzipitalplatte befestigt ist, die mit dem Verbindungselement und dem Stabelelement verschraubt ist,
Figur 9 einen Schnitt durch den menschlichen Schädel mit Sicht auf das Hinterhauptbein mit Hinterhauptsloch von innen, in das das Hakenelement der Okzipitalplatte eingreift.

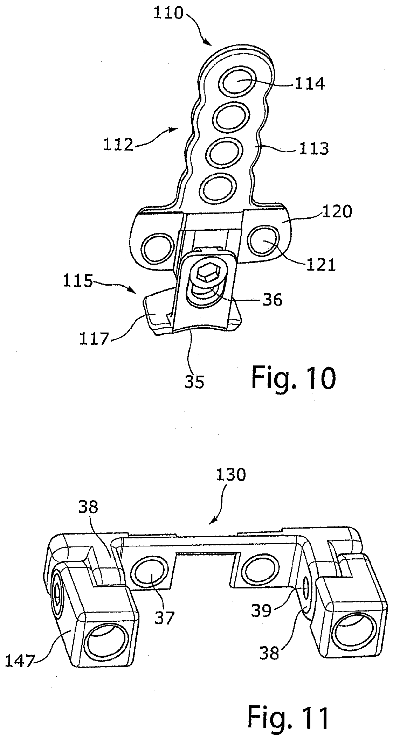

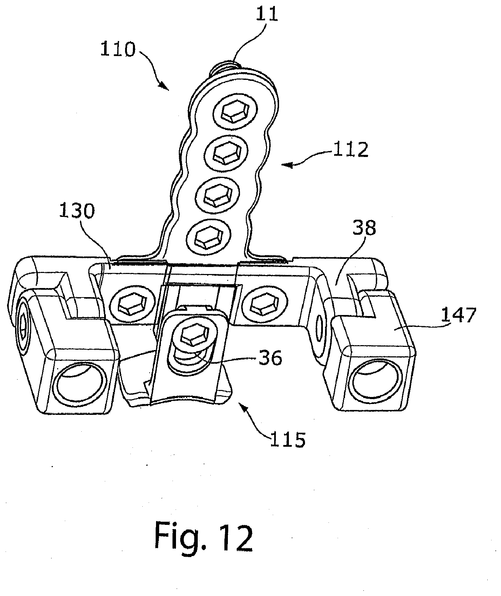

Bei den in den Figuren 10-12 gezeigten Beispielen handelt es sich nicht um Ausführungsbeispiele der Erfindung. Sie dienen lediglich dem Verständnis der Erfindung.

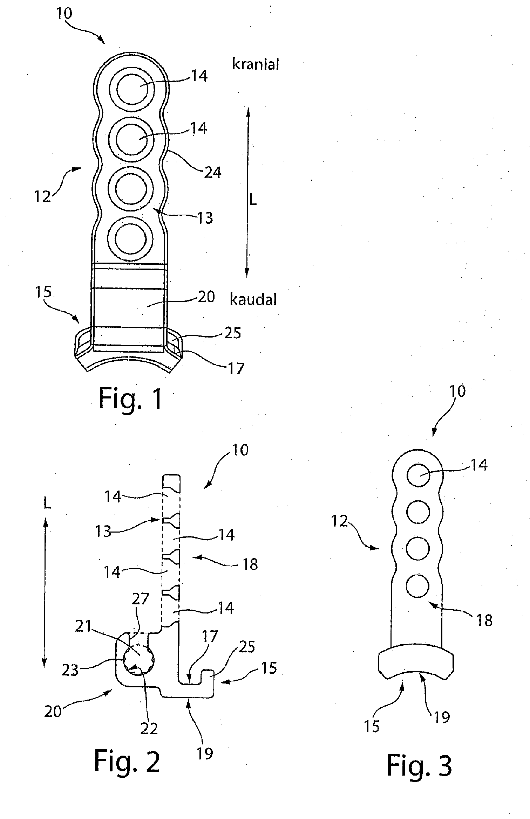

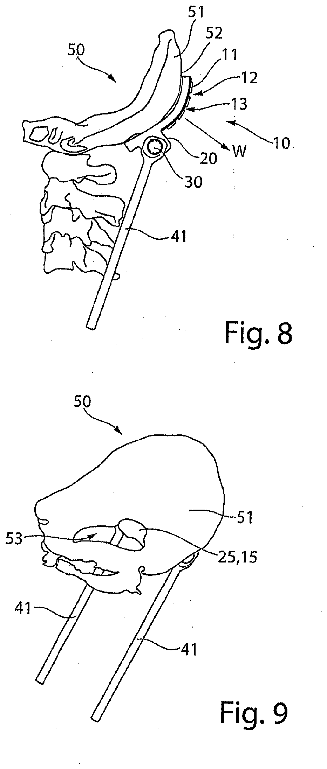

Die Okzipitalplatte 10 in den Figuren 1, 2 und 3 besitzt einen länglichen Mittelabschnitt 12, der am Schädel 50 in kranial-kaudaler Richtung, d.h. in Richtung der Längsachse L in Figur 1, zu befestigen ist. Die Okzipitalplatte 10 weist eine Vorderseite 13 auf und eine Rückseite 18, wobei die Vorderseite 13 parallel zur Rückseite 18 verläuft und dieser gegenüberliegt. Nach der Befestigung am Schädel 50 weist die Rückseite 18 in Richtung des Schädels 50, vgl. Figur 8.

Die Okzipitalplatte 10 weist einen Mittelabschnitt 12 mit vier Durchführungen 14 auf, die als Bohrungen 14 ausgebildet sind. Die Durchführungen 14 sind in der kranialen Richtung des Mittelabschnitts 12 vorgesehen. Die Bohrungen 14 dienen dazu, die Okzipitalplatte 10 mit Schrauben 11 am Schädel 50 zu befestigen. Im Bereich der Durchführungen 14 folgt die Außenkontur 24 des Mittelabschnitts 12 der Außenkontur der Bohrungen 14 bzw. der Köpfe der Schrauben 11 in den Bohrungen 14, d.h. im distalen Bereich des Mittelabschnitts 12 weist die Außenkontur 24 des Mittelabschnitts 12 einen wellenförmigen, mit den Durchführungen 14 korrespondierenden Verlauf auf.

Die Vorder- 13 und Rückseite 18 des Mittelabschnitts 12 sind parallel zueinander, der Mittelabschnitt 12 ist im oberen (in kranialer Richtung) Bereich plattenförmig.

Der Mittelabschnitt 12 ist an sich starr, kann jedoch während der Operation an die jeweilige Schädelform durch Biegeinstrumente angepasst werden.

Im unteren, in kaudale Richtung weisenden Bereich des Mittelabschnitts 12 weist dieser eine sich von der Vorderseite 13 des Mittelabschnitts 12 distal erstreckende Aufnahme 20 mit einer Durchgangsbohrung 21 auf, die zur formschlüssigen Befestigung einer Stützeinrichtung 40 dient. Die Aufnahme 20 ist annähernd quaderförmig und erstreckt sich von der Vorderseite 13 des Mittelabschnitts 12 weg vom Schädel 50 (Pfeil W in Figur 8) nach außen, etwa unter einem rechten Winkel.

Die Längsachse der Durchgangsbohrung 21 in der Aufnahme 20 verläuft in einer zu der Vorderseite 13 des Mittelabschnitts 12 parallel verlaufenden Ebene. Die Längsachse der Durchgangsbohrung 21 verläuft sowohl in etwa rechtwinklig zur Längsachse L des Mittelabschnitts 12, als auch rechtwinklig zu den Längsachsen der Durchführungen 14. Die Durchgangsbohrung 21 weist an ihrer Innenfläche 22 Führungselemente 23 auf. Ausgehend von der Durchgangsbohrung 21 erstreckt sich eine weitere Bohrung 27 nach oben, vgl. Fig. 2. Durch diese weitere Bohrung 27 und eine Madenschraube 28 kann ein seitliches Verschieben des später beschriebenen Verbindungselements 30 verhindert werden.

Unterhalb der Aufnahme 20 ist am in kaudale Richtung weisenden Ende der Rückseite 18 des Mittelabschnitts 12 der Okzipitalplatte 10 ein Hakenelement 15 angeformt. Das Hakenelement 15, die Aufnahme 20 und der Mittelabschnitt 12 sind einstückig ausgebildet.

Das Hakenelement 15 erstreckt sich mit seinem Boden 19 etwa rechtwinklig von der Rückseite 18 des Mittelabschnitts 12 in Richtung Schädel 50. Die nach oben weisende Seite 17 des Bodens 19 bildet die Auflage für den sich oberhalb des Hinterhauptslochs 53 befindlichen Teil des Schädelknochens (Fig. 9). An dem Ende des in Richtung des Schädels 50 gerichteten Bodens 19 kann zusätzlich ein rechtwinklig nach oben verlaufender Steg 25 vorgesehen sein, der zusammen mit der Oberseite 17 des Bodens 19 und der Rückseite 18 des Mittelabschnitts 12 einen U-förmigen Haken bildet. Umgreift der U-förmige Haken durch das Hinterhauptsloch 53 das Hinterhauptbein 51 nun von unten, so wird eine weitere Befestigung zwischen Schädelknochen/Hinterhauptbein 51 und Okzipitalplatte 10 bereitgestellt, die keine Verschraubung erfordert. Durch die Anordnung des Hakenelements 15 und das Unter- oder Umgreifen des Hinterhauptbeins 51 von unten wird eine einfache und sehr stabile zusätzliche Befestigung am Hinterhauptbein 51 ermöglicht, die ohne Schrauben auskommt.

In dieser Variante des Hakenelements 15 mit dem Steg 25 ist die Okzipitalplatte 10 auch gegen ein Verkippen gesichert.

In einer einfacheren Variante weist das Hakenelement 15 keinen Steg 25 auf. Dann stützt sich ein Teil des Hinterhauptbeins 51 auf der Oberseite 17 des Bodens 19 ab.

Wie in Figur 1 ersichtlich, ist der Boden 19 nach oben, d.h. in kraniale Richtung konvex gekrümmt. Die konvexe Wölbung entspricht dem Verlauf des oberen Bereichs des Hinterhauptslochs 53, vgl. Figur 9.

Die Form und Größe des Hakenelements 15 ist selbstverständlich an die Form und Größe des oberhalb des Hinterhauptslochs 53 liegenden Hinterhauptbeins 51 angepasst.

Die Durchführungen 14 verjüngen sich von der Vorderseite 13 hin zur Rückseite 18, damit Schraubenköpfe von Befestigungsmitteln 11 in den Durchführungen 14 versenkt werden können und nicht aus der Vorderseite 13 herausragen.

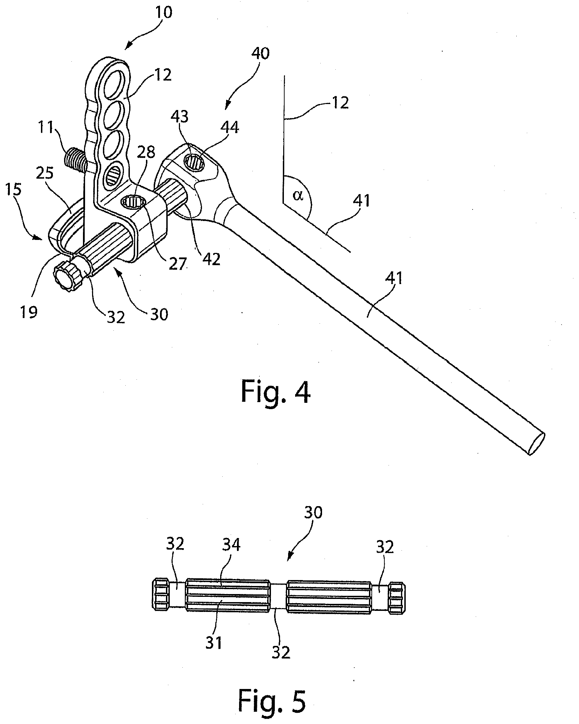

Figur 4 zeigt ein Set mit einer Okzipitalplatte 10, die am Schädel 50 (nicht dargestellt) mittels Schrauben 11 (Befestigungsmittel) befestigt werden kann, und einer Stützeinrichtung 40. Die Stützeinrichtung 40 umfasst Stabelemente 41, die an ihrem distalen Ende an den Wirbeln befestigt werden können und ein Verbindungselement 30, das die Okzipitalplatte 10 mit den Stabelementen 41 verbindet.

In Figur 4 ist zur besseren Erläuterung der Funktion nur ein Stabelement 41 dargestellt.

In der in Figur 4 dargestellten bevorzugten Ausführungsform erfolgt die Verbindung zwischen der Okzipitalplatte 10 und den Stabelementen 41 durch ein Verbindungselement 30, das stabförmig ist.

Das stabförmige Verbindungselement 30 ist in der Durchgangsbohrung 21 in der Aufnahme 20 der Okzipitalplatte 10 befestigbar. Das stabförmige Verbindungselement 30 weist an seiner Mantelfläche 31 im Bereich der Durchgangsbohrung 21 der Aufnahme 20 eine zu den Führungselementen 23 an der Innenfläche 22 der Durchgangsbohrung 21 komplementäre Kontur 34 auf, so dass das stabförmige Verbindungselement 30 in die Durchgangsbohrung 21 der Aufnahme 20 hineingesteckt werden kann und eine Rotationsbewegung des stabförmigen Verbindungselements 30 gegenüber der Okzipitalplatte 10 gesperrt ist.

Die zu den Führungselementen 23 an der Innenfläche 22 der Durchgangsbohrung 21 der Aufnahme 20 komplementären Konturen 34 auf der Mantelfläche 31 des stabförmigen Verbindungselements 30 sind durch umlaufende Nuten 32 unterbrochen. In die Nuten 32, die sich mittig und nahe den beiden Enden des Verbindungselements 30 befinden, können Schrauben 28, 43 in die entsprechenden Bohrungen 27 in der Aufnahme 20 der Okzipitalplatte 10 und die Bohrungen 44 an den proximalen Enden der Stabelemente 41 eingeschraubt werden, so dass ein seitliches Verschieben des stabförmigen Verbindungselements 30 gegenüber der Durchgangsbohrung 21 in der Aufnahme 20 und gegenüber der später beschriebenen Durchgangsbohrung 42 am proximalen Ende des Stabelements 41 verhindert wird.

Das Verbindungselement 30 kann ein runder Stab, insbesondere eine gezahnte Welle, sein, wobei der Stab auf seiner Mantelfläche 31 eine Kontur 34 aufweist, die zu den Führungselementen 23 auf der Innenfläche 22 der Durchgangsbohrung 21 komplementär ist. Die Kontur 34 kann beispielsweise wellen- oder zackenförmig sein, wie in Figur 5 zu sehen. Die Kontur 34 erstreckt sich über die gesamte Mantelfläche 31 des Verbindungselements 30. Das Verbindungselement 30 ist zylindrisch, so dass es durch die Durchgangsbohrung 21 in der Aufnahme 20 hindurchgesteckt werden kann. Die wellen- oder zackenförmigen oder anderweitig ausgebildeten Konturen 34 auf der Mantelfläche 31 des Verbindungselements 30 sind bezüglich der Rotationsachse des stabförmigen Verbindungselements 30 symmetrisch verteilt, und die Konturen 34 verlaufen entlang der Mantelfläche 31 parallel zueinander.

Das Verbindungselement 30 befindet sich im montierten Zustand in der Durchgangsbohrung 21 der Aufnahme 20. Aufgrund des Ineinandergreifens der Kontur 34 auf der Mantelfläche 31 und der Führungselemente 23 auf der Innenfläche 22 sind somit die Okzipitalplatte 10 und das Verbindungselement 30 winkelstabil verbunden, d.h., dass das stabförmige Verbindungselement 30 in der Durchgangsbohrung 21 nicht drehbar ist. Wie in Figur 4 dargestellt, wird das Verbindungselement 30 mit einer Madenschraube 28 auch gegen seitliches Verschieben gesichert. Die Madenschraube 28 ist durch eine Bohrung 27 mit einem Innengewinde, das rechtwinklig zu der Durchgangsbohrung 21 verläuft, in die Aufnahme 20 geschraubt und greift mit ihrer Spitze in die mittige Ringnut 32 im Verbindungselement 30 (Figur 5).

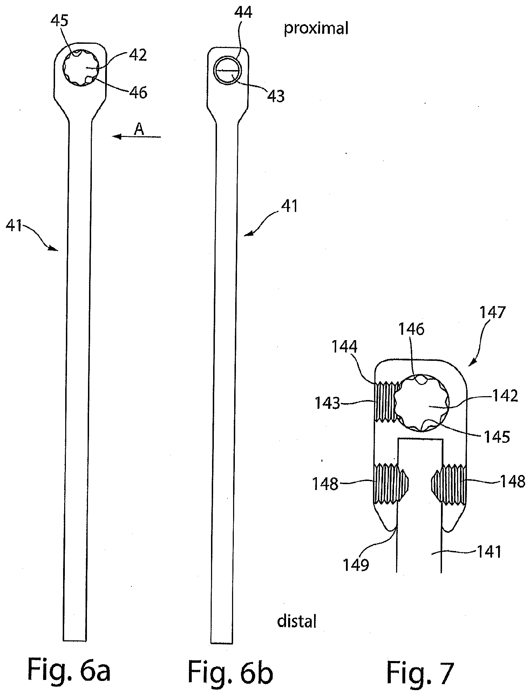

Figur 6a zeigt das Stabelement 41 in einer Seitenansicht. Das proximale Ende des Stabelements 41 ist verbreitert, um eine bessere Kopplung mit dem Verbindungselement 30 zu ermöglichen. Das proximale Ende weist ebenfalls eine Durchgangsbohrung 42 auf, die an ihrer Innenfläche 45 Führungselemente 46 aufweist, die entsprechend den Führungselementen 23 in der Aufnahme 20 ausgebildet sein können, wie bereits zuvor bei der Innenfläche 22 der Durchgangsbohrung 21 der Aufnahme 20 beschrieben. Analog zur Aufnahme 20 kann das Verbindungselement 30 mit seinem Endbereich in die Bohrung 42 in dem proximalen Ende des Stabelements 41 gesteckt werden, so dass das Verbindungselement 30 formschlüssig mit dem Stabelement 41 verbunden ist. Wie bereits zuvor beschrieben, wird das Stabelement 41 zudem mit dem Verbindungselement 30 durch eine Madenschraube 43 in der Bohrung 44 am proximalen Ende des Stabelements 41 verschraubt, um eine seitliche Verschiebebewegung des Verbindungselements 30 gegenüber dem Stabelement 41 zu verhindern.

Das Stabelement 41 wird gerade so weit auf das Verbindungselement 30 gesteckt, dass deren Außenflächen bündig sind.

Somit besteht auch zwischen Verbindungselement 30 und dem Stabelement 41 eine winkelstabile Verbindung.

Durch die formschlüssigen Verbindungen zwischen den beiden Enden des stabförmigen Verbindungselements 30 mit dem proximalen Ende der beiden Stabelemente 41 und der formschlüssigen Verbindung zwischen dem stabförmigen Verbindungselement 30 und der Okzipitalplatte 10 und die durch die Führungselemente 23, 46 und korrespondierenden Konturen 34 bereitgestellten diskreten Eingriffspositionen ist der Winkel zwischen den Stabelementen 41 und der Okzipitalplatte 10 - entsprechend dem Winkel des Kopfes gegenüber der Halswirbelsäule und ohne manuelle Biegung der Stabelemente 41 - einstellbar. Darüber hinaus ist die Verbindung durch diese Art der Koppelung zudem sehr winkelstabil, was bei den aus dem Stand der Technik bekannten einfachen Schraubverbindungen zwischen Okzipitalplatte 10 und Stabelementen 41 nicht der Fall ist.

Die Draufsicht in Figur 6b zeigt die Bohrung 44 im Stabelement 41 mit der Schraube 43, die rechtwinklig zu der Durchgangsbohrung 42 ist. Wie bereits zuvor beschrieben, wird das Stabelement 41 mit dem Verbindungselement 30 durch die Madenschraube 43 in der Bohrung 44 im proximalen Ende des Stabelements 41 verschraubt, um eine seitliche Verschiebebewegung des Verbindungselements 30 gegenüber dem Stabelement 41 zu verhindern.

Figur 7 zeigt eine alternative Ausführungsform des proximalen Endes eines Stabelements 141. Das proximale Ende des Stabelements 141 ist in dieser Ausführungsform nicht verbreitert. Das Stabelement 141 weist über seine gesamte Länge einen gleichbleibenden Durchmesser auf. An seinem proximalen Ende ist ein Kopplungselement 147 befestigt, das das Stabende in einer inneren Stabaufnahme 149 aufnimmt. Bis auf eine Befestigung 148 zwischen Kopplungselement 147 und Ende des Stabelements 141 ist das Ende des Stabelements 141 analog zu dem proximalen Ende des Stabelements 41 aus Figur 8 ausgebildet und weist eine Durchgangsbohrung 142 und eine Bohrung 144 auf. Wie bereits zuvor beschrieben, wird auch das Kopplungselement 147 mit dem Verbindungselement 30 durch eine Madenschraube 143 in der Bohrung 144 im Kopplungselement 147 verschraubt, um eine seitliche Verschiebebewegung des Verbindungselements 30 gegenüber dem Stabelement 141 zu verhindern.

Das Stabelement 141 ist mit zwei Schrauben 148 gegen Herauslösen aus der Stabaufnahme 149 gesichert.

Im Rahmen der vorliegenden Erfindung umfasst der Begriff "Stabelement mit einer Durchgangsbohrung" auch ein Stabelement 141, an dessen proximalem Ende ein Kopplungselement 147 mit einer Durchgangsbohrung 142 vorgesehen ist.

Die Okzipitalplatte 10 aus Figur 1 bis 3 ist in Figur 8 und Figur 9 mit vier Schrauben 11 im Bereich des Hinterhauptbeins (Okziput) 51 am Schädel 50 befestigt. Der Mittelabschnitt 12 ist in Längsrichtung L mit einem Biegeinstrument in die der Wölbung des Hinterhauptbeins 51 entsprechende Form gebogen. Das U-förmige Hakenelement 15 umgreift durch das große Hinterhauptsloch (Foramen magnum) 53 den Schädelknochen von unten. Die Okzipitalplatte 10 ist durch den Steg 25 auch gegen ein Verkippen gesichert.

Das Hinterhauptbein 51 ist mittig entlang eines sich in Längsrichtung L erstreckenden Bereichs 52 verdickt.

Der Mittelabschnitt 12 ist so auf dem Hinterhauptbein 51 positioniert, dass die Durchführungen 14 über dem Bereich 52 liegen und somit die Befestigungsmittel 11 in diesen dickeren und stärkeren Bereich 52 des Schädels 50 geschraubt werden können.

Die Stabelemente 41 werden vom Operateur an den Verlauf der Wirbelsäule angepasst, anschließend im gewünschten Winkel auf das Verbindungselement 30 geschoben und mit den Madenschrauben 43 fixiert. Anschließend werden die Stabelemente 41 mit den Wirbelkörpern verschraubt.

## Patentansprüche

1. Okzipitalplatte (10) zur Befestigung am menschlichen Schädel (50), wobei die Okzipitalplatte (10) einen Mittelabschnitt (12) mit wenigstens einer Durchführung (14) für wenigstens ein Befestigungsmittel (11) zum Befestigen am Schädel (50) aufweist und wenigstens eine Aufnahme (20) für eine Stützeinrichtung (40), wobei die wenigstens eine Aufnahme (20) starr mit dem Mittelabschnitt (12) verbunden ist, wobei
die Okzipitalplatte (10) an ihrem in kaudale Richtung weisenden Ende wenigstens ein Hakenelement (15) aufweist, das dazu dient, in dem Hinterhauptsloch (53) eingehakt zu werden, wobei die wenigstens eine Aufnahme (20) eine Durchgangsbohrung (21) zur Verbindung mit der Stützeinrichtung (40) aufweist,
**dadurch gekennzeichnet,**
**dass** die Längsachse der Durchgangsbohrung (21) in der wenigstens einen Aufnahme (20) in einer zu der Vorderseite (13) des Mittelabschnitts (12) parallel liegenden Ebene und rechtwinklig zur Längsachse L des Mittelabschnitts (12) verläuft und der Mittelabschnitt (12), die wenigstens eine Aufnahme (20) und das Hakenelement (15) einstückig sind.

2. Okzipitalplatte (10) nach Anspruch 1, **dadurch gekennzeichnet, dass** das Hakenelement (15) in Richtung des kranialen Endes des Mittelabschnitts (12) konvex gekrümmt ist.

3. Okzipitalplatte (10) nach einem der vorangegangenen Ansprüche, **dadurch gekennzeichnet, dass** sich die wenigstens eine Aufnahme (20) von der Vorderseite (13) des Mittelabschnitts (12) weg vom Schädel (50) erstreckt.

4. Set zum Fixieren des Schädels (50) an der Wirbelsäule, umfassend eine Okzipitalplatte (10) nach einem der Ansprüche 1 bis 3, eine mit der Okzipitalplatte (10) verbindbare Stützeinrichtung (40), die Stabelemente (41, 141) und Verbindungselemente (30) und/oder Befestigungsmittel (28, 43) zur starren Verbindung der Stützeinrichtung (40) mit der Okzipitalplatte (10) aufweist.

5. Set nach Anspruch 4, **dadurch gekennzeichnet, dass** das Verbindungselement (30) stabförmig ist und mit der Okzipitalplatte (10) und den Stabelementen (41, 141) formschlüssig und winkelstabil verbindbar ist.

6. Set nach Anspruch 4 oder 5, **dadurch gekennzeichnet, dass** die Stabelemente (41) an oder in ihrem proximalen Ende Durchgangsbohrungen (42) mit Führungselementen (46) an ihrer Innenfläche (45) zur Befestigung des Verbindungselements (30) aufweisen.

7. Set nach einem der Ansprüche 4 bis 6, **dadurch gekennzeichnet, dass** das stabförmige Verbindungselement (30) auf seiner Mantelfläche (31) Konturen (34) aufweist, die zu den Führungselementen (23, 46) auf der Innenfläche (22) der Durchgangsbohrung (21) der wenigstens einen Aufnahme (20) der Okzipitalplatte (10) und der Innenfläche (45) der Durchgangsbohrung (42) der Stabelemente (41) komplementär sind, so dass die Führungselemente (23, 46) in die komplementären Konturen (34) auf der Mantelfläche (31) des Verbindungselements (30) eingreifen können.

8. Set nach einem der Ansprüche 4 bis 7, **dadurch gekennzeichnet, dass** das stabförmige Verbindungselement (30) in der Durchgangsbohrung (21) der wenigstens einen Aufnahme (20) und der Durchgangsbohrung (42) des Stabelements (41) arretierbar ist.

## Claims

1. Occipital plate (10) for attachment to the human skull (50), wherein the occipital plate (10) has a central portion (12) with at least one passage (14) for at least one attachment means (11) for attachment to the skull (50) and at least one receptacle (20) for a support device (40), wherein the at least one receptacle (20) is rigidly connected to the central portion (12), wherein
the occipital plate (10) has, at its end pointing in the caudal direction, at least one hook element (15) which serves to be hooked into the occipital hole (53), the at least one receptacle (20) having a through-hole (21) for connection to the support device (40),
**characterized in**
**that** the longitudinal axis of the through-hole (21) in the at least one receptacle (20) extends in a plane parallel to the front side (13) of the central section (12) and at right angles to the longitudinal axis L of the central section (12), and the central section (12), the at least one receptacle (20) and the hook element (15) are in one piece.

2. Occipital plate (10) according to claim 1, **characterized in that** the hook element (15) is convexly curved in the direction of the cranial end of the central portion (12).

3. Occipital plate (10) according to one of the preceding claims, **characterized in that** the at least one receptacle (20) extends from the front side (13) of the middle section (12) away from the skull (50).

4. Set for fixing the skull (50) to the spinal column, comprising an occipital plate (10) according to one of claims 1 to 3, a support device (40) which can be connected to the occipital plate (10), which support device has rod elements (41, 141) and connecting elements (30) and/or fastening means (28, 43) for rigidly connecting the support device (40) to the occipital plate (10).

5. Set according to claim 4, **characterized in that** the connecting element (30) is rod-shaped and can be connected to the occipital plate (10) and the rod elements (41, 141) in a form-fitting and angularly stable manner.

6. Set according to claim 4 or 5, **characterized in that** the rod elements (41) have through-holes (42) at or in their proximal end with guide elements (46) on their inner surface (45) for fastening the connecting element (30).

7. Set according to one of claims 4 to 6, **characterized in that** the rod-shaped connecting element (30) has contours (34) on its outer surface (31) which are complementary to the guide elements (23, 46) on the inner surface (22) of the through-hole (21) of the at least one receptacle (20) of the occipital plate (10) and to the inner surface (45) of the through-hole (42) of the rod elements (41), so that the guide elements (23, 46) can engage in the complementary contours (34) on the lateral surface (31) of the connecting element (30).

8. Set according to one of claims 4 to 7, **characterized in that** the rod-shaped connecting element (30) can be locked in the through-hole (21) of the at least one receptacle (20) and the through-hole (42) of the rod element (41).

## Revendications

1. Set occipitale (10) destinée à être fixée au crâne humain (50), la plaque occipitale (10) comprenant une partie centrale (12) avec au moins un passage (14) pour au moins un moyen de fixation (11) destiné à être fixé au crâne (50) et au moins un logement (20) pour un dispositif de support (40), ledit au moins un logement (20) étant relié de manière rigide à la partie centrale (12), dans laquelle
la plaque occipitale (10) comporte, à son extrémité orientée dans la direction caudale, au moins un élément de crochet (15) destiné à être accroché dans le trou occipital (53), ledit au moins un logement (20) comportant un trou traversant (21) destiné à être relié au dispositif de support (40), **caractérisé en ce que**
l'axe longitudinal du trou traversant (21) dans ledit au moins un logement (20) s'étend dans un plan parallèle à la face avant (13) de la section centrale (12) et perpendiculaire à l'axe longitudinal L de la section centrale (12), et la section centrale (12), ledit au moins un logement (20) et l'élément d'accrochage (15) sont d'une seule pièce.

2. Set occipitale (10) selon la revendication 1, **caractérisée en ce que** l'élément de crochet (15) est incurvé de manière convexe en direction de l'extrémité crânienne de la section centrale (12).

3. Set occipitale (10) selon l'une des revendications précédentes, **caractérisée en ce que** ledit au moins un logement (20) s'étend depuis la face avant (13) de la portion centrale (12) en s'éloignant du crâne (50) .

4. Set de fixation du crâne (50) à la colonne vertébrale, comprenant une plaque occipitale (10) selon l'une quelconque des revendications 1 à 3, un dispositif de support (40) pouvant être relié à la plaque occipitale (10) et comprenant des éléments de tige (41, 141) et des éléments de liaison (30) et/ou des moyens de fixation (28, 43) pour relier rigidement le dispositif de support (40) à la plaque occipitale (10).

5. Set selon la revendication 4, **caractérisé en ce que** l'élément de liaison (30) est en forme de tige et peut être relié à la plaque occipitale (10) et aux éléments de tige (41, 141) par complémentarité de forme et avec une stabilité angulaire.

6. Set selon la revendication 4 ou 5, **caractérisé en ce que** les éléments de tige (41) présentent à ou dans leur extrémité proximale des trous traversants (42) avec des éléments de guidage (46) sur leur surface intérieure (45) pour la fixation de l'élément de liaison (30).

7. Set selon l'une des revendications 4 à 6, **caractérisé en ce que** l'élément de liaison (30) en forme de tige présente sur sa surface latérale (31) des contours (34) qui sont complémentaires aux éléments de guidage (23, 46) sur la face interne (22) du trou traversant (21) de l'au moins un logement (20) de la plaque occipitale (10) et à la face interne (45) du trou traversant (42) des éléments de tige (41), de sorte que les éléments de guidage (23, 46) peuvent s'engager dans les contours complémentaires (34) sur la face latérale (31) de

8. Set selon l'une des revendications 4 à 7, **caractérisé en ce que** l'élément de liaison (30) en forme de tige peut être arrêté dans le trou traversant (21) dudit au moins un logement (20) et dans le trou traversant (42) de l'élément de tige (41) .
